# EUROPEAN PATENT APPLICATION

(11) **EP 3 988 643 A1**
(43) Date of publication of application: **27.04.2022**
(21) Application number: 21204568.6
(22) Date of filing: 25.10.2021
(51) Int. Cl.: C12N 1/20, C12P 7/24

(54) **METHOD FOR PREPARING NATURAL VANILLIN VIA BIOOXIDATION OF 4-METHYLGUAIACOL**

(30) Priority: 23.10.2020 CN 202011152128
(71) Applicant: Xiamen Oamic Biotech Co., Ltd., Xiamen, Fujian 361026 (CN)
(72) Inventor: ZENG, Shichao, Xiamen, 361026 (CN); ZHAO, Xiaolan, Xiamen, 361026 (CN); GUO, Yashan, Xiamen, 361026 (CN); LIN, Xiaohui, Xiamen, 361026 (CN); CAI, Haifang, Xiamen, 361026 (CN); LIU, Chaoxia, Xiamen, 361026 (CN); XING, Chenguang, Xiamen, 361026 (CN); LIU, Gang, Xiamen, 361026 (CN)
(74) Representative: Verscht, Thomas Kurt Albert

(57) **Abstract**

A method for preparing natural vanillin via biooxidation of 4-methylguaiacol comprises (1) activating a strain and preparing a seed culture solution; (2) fermenting; (3) bioconversion; (4) adsorbing and eluting; and (5) concentrating and crystallizing. The present disclosure provides a biooxidation method for preparing natural vanillin using 4-methylguaiacol as a raw material, and novel routes are available for the preparation of the nature vanillin. The *Bacillus megaterium* OMK-72 of the present disclosure is used to ferment and oxidize 4-methylguaiacol to prepare vanillin. The concentration of the vanillin in the conversion solution can reach 16.2 g/L, a production cost is low, and industrial application prospects are good.

## Description

### FIELD OF THE DISCLOSURE

The present disclosure relates to a technical field of fermentation engineering, and in particular relates to a method for preparing natural vanillin via biooxidation of 4-methylguaiacol.

### BACKGROUND OF THE DISCLOSURE

Vanillin is an important flavor and fragrance material extracted from vanilla beans of rutaceae plants, which has the strong and unique aroma of the vanilla beans has one of the largest share in the flavor and fragrance output, and is an important raw material for preparing chocolate, ice cream, chewing gum, cakes, and tobacco flavors.

At present, the vanillin on the market is mainly obtained from chemical synthesis, such as various methods including the lignin method or the guaiacol-glyoxylic acid method. However, as consumers pursue a healthier and more environmentally friendly life style with the development of society, market share of natural flavors and fragrances gets higher and higher. Production of the vanillin by developing a purely natural biological process has become a hot spot of research and development. Existing methods for preparing natural vanillin mainly comprise the ferulic acid route or the eugenol route.

### BRIEF SUMMARY OF THE DISCLOSURE

An objective of the present disclosure is to provide a method for preparing natural vanillin via biooxidation of 4-methylguaiacol.

Another objective of the present disclosure is to provide *Bacillus megaterium* OMK-72 used in the aforementioned method.

A technical solution of the present disclosure is as follows.

A method for preparing natural vanillin via biooxidation of 4-methylguaiacol comprises the following steps.
(1) culturing *Bacillus megaterium* OMK-72 on a solid slant medium and a seed medium in sequence to obtain a seed culture solution, wherein the *Bacillus megaterium* OMK-72 was deposited in the China Center for Type Culture Collection (located in Wuhan University, Wuhan, China) on August 31, 2020, with a deposition number of CCTCC NO: M 2020457;
(2) inoculating the seed culture solution into a fermentation medium and cultivating until the *Bacillus megaterium* OMK-72 grows to an early logarithmic stage, then adding 1.8-2.2 g/L of 4-methylguaiacol to induce an expression of correlated enzymes, and cultivating until fermentation is complete;
(3) adding 4-methylguaiacol into a culture solution obtained in step (2) at one time or several times, and fully stirring and reacting under aeration conditions at the same time, wherein during this step, a concentration of the 4-methylguaiacol for each addition is 4.5-5.5 g/L;
(4) centrifuging a material obtained in step (3) to obtain a supernatant, letting the supernatant to pass through a ceramic membrane to obtain a clear solution, letting the clear solution to pass through a resin column for absorption, and then eluting with ethanol to obtain an eluate; and
(5) evaporating and concentrating the eluate, then crystallizing, and then drying to obtain the natural vanillin.

In a preferred embodiment, the method comprises the following steps.
(1) streaking the *Bacillus megaterium* OMK-72 from a cryopreserved glycerol tube (e.g., preserved at a low temperature) on the solid slant medium and then culturing at 28-30 °C for 24-48 hours to obtain an activated strain, and inoculating the activated strain into the seed medium and culturing to the early logarithmic stage at 28-30°C and 200-250 rpm with shaking to obtain the seed culture solution;
(2) inoculating the seed culture solution obtained in step (1) into the fermentation medium with a volume ratio of 5-10% and cultivating for 3-6 hours under conditions of pH 6.5-8.0, 28-30 °C, a stirring speed of 100-600 rpm, and an aeration rate of 1:0.5, when the *Bacillus megaterium* OMK-72 grows to the early logarithmic stage, adding 1.8-2.2 g/L of the 4-methylguaiacol and continuing to ferment for 36-48 hours to obtain the culture solution;
(3) adding 4-methylguaiacol into a culture solution obtained in step (2) at one time or several times, converting under conditions of a stirring speed of 300-600 rpm and an aeration rate of 1:0.5, wherein during this step, a concentration of the 4-methylguaiacol for each addition is 4.5-5.5 g/L;
(4) centrifuging a material obtained in step (3) to obtain a supernatant, letting the supernatant to pass through a ceramic membrane to obtain a clear solution, letting the clear solution to pass through a resin column for absorption, and then eluting with ethanol to obtain an eluate; and
(5) transferring the eluate obtained in step (4) to a distillation flask, evaporating an amount (e.g., an appropriate amount) of the ethanol, concentrating (e.g., appropriately concentrating), crystallizing under a condition (e.g., a appropriate condition), and drying to obtain the natural vanillin.

In a preferred embodiment, a composition of the solid slant medium has the following mass percentages: peptone 1-2%, yeast extract 0.5-1%, sodium chloride 0.5-1%, agar 1.5-2%, and a solvent is water.

In a further preferred embodiment, the composition of the solid slant medium has the following mass percentages: peptone 1%, yeast extract 0.5%, sodium chloride 1%, agar 2%, and the rest is water.

In a preferred embodiment, a composition of the seed medium has the following mass percentages: peptone 1-2%, yeast extract 0.5-1%, sodium chloride 0.5-1%, and a solvent is water.

In a further preferred embodiment, a composition of the seed medium has the following mass percentages: peptone 1%, yeast extract 0.5%, sodium chloride 1%, and the rest is water.

In a preferred embodiment, a composition of the fermentation medium has the following mass percentages: glucose 2.0-5.0%, ammonium dihydrogen phosphate 0.5-1.0%, potassium dihydrogen phosphate 0.1- 0.5%, magnesium sulfate 0.05-0.2%, calcium sulfate 0.05-0.1%, yeast extract powder 0.1-1.0%, trace element solution 0.1-0.2%, and a solvent of the fermentation medium is water; and a composition of the trace element solution has the following mass percentages: ethylenediaminetetraacetic acid (EDTA) 0.8 -1.2%, zinc sulfate 0.1-0.3%, calcium chloride 0.08-0.12%, ferrous sulfate 0.45-0.55%, sodium molybdate 0.01-0.03%, copper sulfate 0.01-0.03%, cobalt chloride 0.03-0.05%, manganese chloride is 0.08-0.12%, and a solvent of the trace element solution is water.

In a further preferred embodiment, a composition of the fermentation medium has the following mass percentages: glucose 2%, ammonium dihydrogen phosphate 1%, potassium dihydrogen phosphate 0.5%, magnesium sulfate 0.2%, calcium sulfate 0.1%, yeast extract powder 0.5%, trace element solution 0.1%, and the rest is water; and the trace element solution has the following mass percentages: ethylenediaminetetraacetic acid (EDTA) 1%, zinc sulfate 0.2%, calcium chloride 0.1%, ferrous sulfate 0.5%, sodium molybdate 0.02%, copper sulfate 0.02%, cobalt chloride 0.04%, manganese chloride 0.1%, and a solvent of the trace element solution is water.

Another technical solution of the present disclosure is as follows.

*Bacillus megaterium* OMK-72 deposited at the China Center for Type Culture Collection (located in Wuhan University, Wuhan, China) on August 31, 2020, with a deposit number of CCTCC NO: M 2020457.

A method for preparing natural vanillin via biooxidation of 4-methylguaiacol using the *Bacillus megaterium* OMK-72 .

The present disclosure has the following advantages.
1. The present disclosure provides a biooxidation method for preparing natural vanillin using 4-methylguaiacol as a raw material, so that the preparation of the natural vanillin no longer completely depends on ferulic acid and eugenol, but with novel route available for the preparation of the nature vanillin.
2. The *Bacillus megaterium* OMK-72 of the present disclosure is used to ferment and oxidize 4-methylguaiacol to prepare vanillin. The concentration of the vanillin in the conversion solution can reach 16.2 g/L, a production cost is low, and industrial application prospects are good.

### BRIEF DESCRIPTION OF THE DRAWINGS

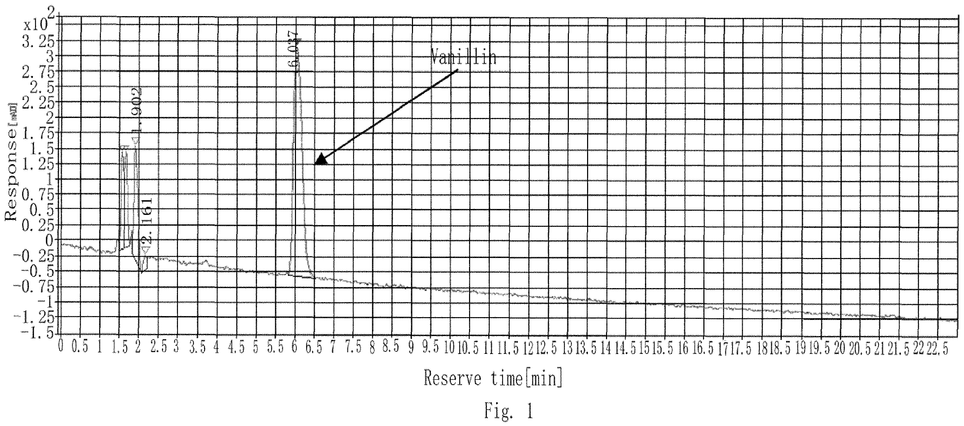

Fig. 1 illustrates the high performance liquid chromatography (HPLC) graph of vanillin of Embodiment 2 of the present disclosure, produced by fermentation and oxidation of 4-methylguaiacol using *Bacillus megaterium* OMK-72.

### DETAILED DESCRIPTION OF THE EMBODIMENTS

The present disclosure will be further described in combination with the accompanying embodiments and drawings.

### Embodiment 1: Preparation of natural vanillin (fermentation using triangular shaking flask)

(1) Acquisition of a strain
   The strain used to catalyze 4-methylguaiacol to produce vanillin is isolated from marine sediments, and is identified as *Bacillus megaterium* by 16S ribosomal deoxyribonucleic acid (rDNA) sequencing and named as *Bacillus megaterium* OMK-72. The strain was deposited in the China Center for Type Culture Collection (located in Wuhan University, Wuhan, China) on August 31, 2020, with the deposit number: CCTCC NO: M 2020457.
(2) Activation of the strain: *Bacillus megaterium* OMK-72 from a glycerol tube at -80 °C is streaked to be spread on a solid slant medium and is left to stand to be cultured at 30 °C for 24 hours, and an activated strain is obtained.
   The component of the solid slant medium has the following mass percentages: peptone 1%, yeast extract 0.5%, sodium chloride 1%, agar 2%, and the rest is water.
(3) Preparation of a seed culture solution: the activated strain is inoculated into a 500 mL triangular shaking flask containing 30 mL of a seed medium and is cultivated at 30 °C and 250 revolutions per minute (rpm) for 4-6 hours to obtain the seed culture solution.
   The component of the seed medium has the following mass percentages: peptone 1%, yeast extract 0.5%, sodium chloride 1%, and the rest is water. The initial pH is 7.5, and the seed medium is sterilized at 121 °C for 20 minutes.
(4) Fermentation and conversation: 5 mL of the seed culture solution is inoculated into a 500 mL triangular shaking flask containing 50 mL of a fermentation medium and is fermented at 30 °C and 250 rpm for 4 hours. After the strain grows to a logarithmic growth phase, 2 g/L of 4-methylguaiacol is added, and the strain is let to continue fermenting at 30 °C and 250 rpm for 36 hours. After the fermentation is complete, 5 g/L of 4-methylguaiacol is added to be converted for 10 hours to achieve complete conversion to obtain a fermentation broth. The concentration of the vanillin in the fermentation broth is 5.4 g/L and a yield of 98% is achieved as measured by a HPLC analysis.

The composition of the fermentation medium has the following mass percentages: glucose 2%, ammonium dihydrogen phosphate 1%, potassium dihydrogen phosphate 0.5%, magnesium sulfate 0.2%, calcium sulfate 0.1%, yeast extract powder 0.5%, trace element solution 0.1%, and the rest is water. The initial pH is 7.5, and the fermentation medium is sterilized at 115 °C for 30 minutes, wherein the trace element solution has the following mass percentages: ethylenediaminetetraacetic acid (EDTA) 1%, zinc sulfate 0.2%, calcium chloride 0.1%, ferrous sulfate 0.5%, sodium molybdate 0.02%, copper sulfate 0.02%, cobalt chloride 0.04%, manganese chloride 0.1%, and the solvent is water.

### Embodiment 2: Preparation of natural vanillin (i.e., vanillin) (fermentation using a fermentation tank)

Steps (1) to (2) are the same as Embodiment 1.
(3) Preparation of a seed culture solution: the activated strain is inoculated into a 500 mL triangular shaking flask containing 50 mL of a seed medium and is cultivated at 30 °C and 250 rpm for 6-8 hours to obtain a first stage seed culture solution; 50 mL of the first stage seed culture solution is completely inoculated into a 10 L seed tank containing 6 L of the seed medium and is cultivated at 30 °C for 6-8 hours with a stirring speed of 250 rpm and an aeration rate of 1:0.5 to obtain a second stage seed culture solution.
   The composition of the seed medium is the same as Embodiment 1.
(4) Fermentation and conversion: 15 L of a fermentation medium is added into a 30 L fermenter and is sterilized at 115 °C for 30 minutes, and the second stage seed culture solution is inoculated into the 30 L fermenter to be cultivated with 10% inoculum volume. The cultivation condition is as follows: the temperature is 30 °C, the stirring speed is 400 rpm, the aeration ratio is 1:0.5. After the fermentation lasts for 5 hours, 2 g/L of 4-methylguaiacol is added and continues to be fermented, and 70% of a glucose solution is added at a flow rate of 20 g/h until the fermentation is complete. The total fermentation duration is 48 hours. After the fermentation is complete, 5 g/L of the 4-methylguaiacol is added for the conversion with a preset stirring speed of 400 rpm and an aeration ratio of 1:0.5, and 5 g/L of 4-methylguaiacol is then respectively supplemented at 6 hours and 15 hours. The total conversion time is 26 hours, the conversion rate for 4-methylguaiacol reaches 100%, the vanillin concentration of the conversion solution is 16.2 g/L, and the yield is 98%. (The HPLC graph is shown in FIG. 1, and a characteristic peak of the natural vanillin is referenced by the arrow).
   The composition of the fermentation medium is the same as Embodiment 1.
(5) Adsorption and elution by a resin column: after the conversion is complete, the temperature is increased to 60 °C for inactivation of the strain, then the conversion solution obtained in step (4) is centrifuged to obtain the supernatant. The supernatant is let to pass through a ceramic membrane to remove the insolubles to obtain a clear solution. The clear solution is let to pass through a resin column for absorption and is then eluted with ethanol to obtain an ethanol solution containing the vanillin (e.g., one of eluates).
(6) Concentration and crystallization: the ethanol solution containing the vanillin obtained in step (5) is transferred into a distillation flask to be evaporated to remove an appropriate amount of ethanol to be appropriately concentrated, crystallized under appropriate conditions, and dried to obtain 253 g of the pure natural vanillin. The yield is 88%.

The present invention may be summarized as follows: A method for preparing natural vanillin via biooxidation of 4-methylguaiacol comprises (1) activating a strain and preparing a seed culture solution; (2) fermenting; (3) bioconversion; (4) adsorbing and eluting; and (5) concentrating and crystallizing. The present disclosure provides a biooxidation method for preparing natural vanillin using 4-methylguaiacol as a raw material, and novel routes are available for the preparation of the nature vanillin. The Bacillus megaterium OMK-72 of the present disclosure is used to ferment and oxidize 4-methylguaiacol to prepare vanillin. The concentration of the vanillin in the conversion solution can reach 16.2 g/L, a production cost is low, and industrial application prospects are good.

The aforementioned embodiments are merely some embodiments of the present disclosure, and the scope of the disclosure is not limited thereto. Thus, it is intended that the present disclosure cover any modifications and variations of the presently presented embodiments provided they are made without departing from the appended claims and the specification of the present disclosure.

## Claims

1. A method for preparing natural vanillin via biooxidation of 4-methylguaiacol, **characterized in that**: the method comprises the following steps:
(1) culturing *Bacillus megaterium* OMK-72 on a solid slant medium and a seed medium in sequence to obtain a seed culture solution, wherein the *Bacillus megaterium* OMK-72 was deposited in the China Center for Type Culture Collection on August 31, 2020, with a deposition number of CCTCC NO: M 2020457;
(2) inoculating the seed culture solution into a fermentation medium and cultivating until the *Bacillus megaterium* OMK-72 grows to an early logarithmic stage, then adding 1.8-2.2 g/L of 4-methylguaiacol to induce an expression of correlated enzymes, and cultivating until fermentation is complete;
(3) adding 4-methylguaiacol into a culture solution obtained in step (2) at one time or several times, and fully stirring and reacting under aeration conditions at the same time, wherein during this step, a concentration of the 4-methylguaiacol for each addition is 4.5-5.5 g/L;
(4) centrifuging a material obtained in step (3) to obtain a supernatant, letting the supernatant to pass through a ceramic membrane to obtain a clear solution, letting the clear solution to pass through a resin column for absorption, and then eluting with ethanol to obtain an eluate; and
(5) evaporating and concentrating the eluate, then crystallizing, and then drying to obtain the natural vanillin.

2. The method according to claim 1, **characterized in that**: the method comprises the following steps:
(1) streaking the *Bacillus megaterium* OMK-72 from a cryopreserved glycerol tube on the solid slant medium and then culturing at 28-30 °C for 24-48 hours to obtain an activated strain, and inoculating the activated strain into the seed medium and culturing to the early logarithmic stage at 28-30°C and 200-250 rpm with shaking to obtain the seed culture solution;
(2) inoculating the seed culture solution obtained in step (1) into the fermentation medium with a volume ratio of 5-10% and cultivating for 3-6 hours under conditions of pH 6.5-8.0, 28-30 °C, a stirring speed of 100-600 rpm, and an aeration rate of 1:0.5, when the *Bacillus megaterium* OMK-72 grows to the early logarithmic stage, adding 1.8-2.2 g/L of the 4-methylguaiacol and continuing to ferment for 36-48 hours to obtain the culture solution;
(3) adding 4-methylguaiacol into a culture solution obtained in step (2) at one time or several times, converting under conditions of a stirring speed of 300-600 rpm and an aeration rate of 1:0.5, wherein during this step, a concentration of the 4-methylguaiacol for each addition is 4.5-5.5 g/L;
(4) centrifuging a material obtained in step (3) to obtain a supernatant, letting the supernatant to pass through a ceramic membrane to obtain a clear solution, letting the clear solution to pass through a resin column for absorption, and then eluting with ethanol to obtain an eluate; and
(5) transferring the eluate obtained in step (4) to a distillation flask, evaporating an amount of the ethanol, concentrating, crystallizing under a condition, and drying to obtain the natural vanillin.

3. The method according to claim 1 and/or 2, **characterized in that**: a composition of the solid slant medium has the following mass percentages: peptone 1-2%, yeast extract 0.5-1%, sodium chloride 0.5-1%, agar 1.5-2%, and a solvent is water.

4. The method according to any one or more of claims 1 to 3, **characterized in that**: a composition of the solid slant medium has the following mass percentages: peptone 1%, yeast extract 0.5%, sodium chloride 1%, agar 2%, and the rest is water.

5. The method according to any one or more of claims 1 to 4, **characterized in that**: a composition of the seed medium has the following mass percentages: peptone 1-2%, yeast extract 0.5-1%, sodium chloride 0.5-1%, and a solvent is water.

6. The method according to any one or more of claims 1 to 5, **characterized in that**: a composition of the seed medium has the following mass percentages: peptone 1%, yeast extract 0.5%, sodium chloride 1%, and the rest is water.

7. The method according to any one or more of claims 1 to 6, **characterized in that**: a composition of the fermentation medium has the following mass percentages: glucose 2.0-5.0%, ammonium dihydrogen phosphate 0.5-1.0%, potassium dihydrogen phosphate 0.1- 0.5%, magnesium sulfate 0.05-0.2%, calcium sulfate 0.05-0.1%, yeast extract powder 0.1-1.0%, trace element solution 0.1-0.2%, and a solvent of the fermentation medium is water; and a composition of the trace element solution has the following mass percentages: ethylenediaminetetraacetic acid 0.8 -1.2%, zinc sulfate 0.1-0.3%, calcium chloride 0.08-0.12%, ferrous sulfate 0.45-0.55%, sodium molybdate 0.01-0.03%, copper sulfate 0.01-0.03%, cobalt chloride 0.03-0.05%, manganese chloride is 0.08-0.12%, and a solvent of the trace element solution is water.

8. The method according to any one or more of claims 1 to 7, **characterized in that**: a composition of the fermentation medium has the following mass percentages: glucose 2%, ammonium dihydrogen phosphate 1%, potassium dihydrogen phosphate 0.5%, magnesium sulfate 0.2%, calcium sulfate 0.1%, yeast extract powder 0.5%, trace element solution 0.1%, and the rest is water; and the trace element solution has the following mass percentages: ethylenediaminetetraacetic acid 1%, zinc sulfate 0.2%, calcium chloride 0.1%, ferrous sulfate 0.5%, sodium molybdate 0.02%, copper sulfate 0.02%, cobalt chloride 0.04%, manganese chloride 0.1%, and a solvent of the trace element solution is water.

9. *Bacillus megaterium* OMK-72 deposited in the China Center for Type Culture Collection on August 31, 2020, with a deposit number of CCTCC NO: M 2020457.

10. A method for preparing natural vanillin via biooxidation of 4-methylguaiacol using the *Bacillus megaterium* OMK-72 according to claim 9.
